# EUROPEAN PATENT APPLICATION

(11) **EP 2 450 114 A1**
(43) Date of publication of application: **09.05.2012**
(21) Application number: 10190388.8
(22) Date of filing: 08.11.2010
(51) Int. Cl.: B09C 1/08, B09C 1/00, C02F 3/34, C11D 17/00

(54) **Gel comprising reactive oxidant release agent**

(71) Applicant: Biorem Engineering SARL, 62780 France (FR)
(72) Inventor: The designation of the inventor has not yet been filed
(74) Representative: Vandeberg, Marie-Paule L.G.

(57) **Abstract**

The invention provides a gel composition comprising 5% to 75% w/w of an inorganic gelling agent other than silica based gelling agents,0.5-60 % w/w, preferably 0.5 to 30 % w/w, of an inorganic oxidant release agent and at least 20 % w/w of water, and possibly a stabilizer. It further provides a process for making same, comprising preparing a first solution of a dibasic phosphate salt in water comprising dissolved oxidant release agent, preparing a second solution of a alkaline earth metal salt in water comprising dissolved oxidant release agent, and mixing both, first and second solutions, in a ratio of 1 to 1 to 10 to 1, followed by a concentration step. The gel composition of the invention is suitable for soil, sediment or (ground)water remediation purposes and disinfection or wound healing purposes.

## Description

### Field of the Invention

The present invention relates to the field of ground water remediation, soil or sediment remediation, water treatment, wound care and disinfection.

### Background to the Invention

In environmental remediation processes, the supply of additional oxygen to contaminated soil and sediments is often used to stimulate the natural aerobic microbial breakdown of contaminants. However, oxygen on itself can also react directly with the pollutants by chemical oxidation. More suitable for this chemical treatment, are stronger oxidizers like hydrogen peroxide, calcium peroxide or persulfate.

Reactive oxidant release agents, such as IXPER 75C (Solvay) or ORC (Regenesis Inc.) are often used in contaminated soil or sediment remediation for their capacity to increase the dissolved oxygen concentration in (ground)water and as such stimulate aerobic processes, such as e.g. microbial processes. They can also directly degrade contaminants by chemical oxidation. In order to convey slow release properties to these release agents, they can be chemically reacted into powdered precipitates, which slowly react with water over time and thus slowly release oxidant in this process.

Existing slow-release agents however have a number of drawbacks, which limit their
applicability in practice. When these agents, for example in the form of calcium or magnesium peroxide, react with water, calcium or magnesium hydroxide is formed, generating a hard solid precipitate. The application of these components into aquifers or submerged sediments is not straightforward, and injection of water-based slurries of these compounds generate clogging problems. Moreover, application of reactive oxidant release agents as a fine powder generates fine particle dust which are irritating to skin and eyes. When hydrogen peroxide solutions are used as oxidant release agent, another problem arises as the injection hereof induces an immediate oxidation reaction of the close environment that cannot be controlled and that can be rather exothermic at higher peroxide concentrations, thus generating vapors and potentially causing volatilization of certain contaminants.

It is thus desirable to provide an improved formulation of oxidants or oxidant release agents for applications in environmental remediation. Such formulation should combine the capacity of oxidant slow-release over time (in contact with water), absence of precipitating reaction products, and user-friendly application.

US2002/0187007 (Schindler) discloses a method for remediating a contaminated region of a subterranean body of groundwater comprising the injection of substantially pure oxygen or oxygen in liquid form to naturally reduce the
contaminants in the groundwater.

US6193776 (Doetsch et al.) discloses a stabilizer (e.g. water glass) for inorganic
peroxygen compounds to obtain a homogeneous calcium/magnesium peroxide, having a magnesium content of 4.2% to 17% by weight, a calcium content of 30 to 53% by weight, and an active oxygen content of 13 to 18%by weight.

US2003/0114334 (Coccia) claims to stabilize liquid compositions containing peroxides by thickening the liquids with water-soluble or water dispersible polymers.

US2008/0274206 (Lekhram et al.) discloses a stabilized liquid oxygen releasing composition comprising unspecified oxygen donor stabilizing agents and liquid binders. Stannates are often used as stabilizers for hydrogen peroxide, mostly in combination with additional stabilizers and/or chelating agents (see US7169237, Wang et al.) There are also different commercial phosphonates available to stabilize hydrogen peroxide and persulfate. For hydrogen peroxide, also1,10-phenanthroline, 8-hydroxyquinoline, citric acid, nitrilotriacetic acid, and ethylenediaminetetraacetic acid have a stabilizing effect (see US4981662, Dougherty, and US7632523, Ramirez et al.).

As the released oxidant is consumed by reaction with surrounding organic matter, an inorganic matrix is preferable over an organic matrix to deliver the oxidizing compounds. However, known peroxide-based gels are mostly based on organic substances like glycerin and propylene glycol (see US5698182, Prencipe et al.), polyvinylpyrrolidones (US5945032, Breitenbach et al.), polyacrylic acid thickening agents (see US2004/0079920, Chadwick et al.), olefinically unsaturated carboxylic acid, and acrylate and methacrylate esters (see US7045493, Wang etal.).

Known inorganic gelling agents include fumed silica (US4839157, Mei-King Ng et al.) and Laponite, a synthetic clay (see US2007/0253918, Campanale et al.). Gels comprising such inorganic gelling agents are proposed in dental applications.

### Purpose of the Invention

The aim of the present invention is to overcome the drawbacks of currently existing oxidant release agents for applications in soil treatment, water treatment, sediment treatment, and disinfection such as: (1) clogging of tubing, solidification in injection filters etc. due to precipitation of reaction products at undesirable places (e.g. calcium hydroxide in the case of calcium peroxide), (2) immediate and strongly exothermic reactions upon contact with the organic material in soil and sediments, creating potentially toxic and dangerous fumes, foam and vapors, (3) limited reactivity of the applied oxidant release agents over time, (4) strong increase or decrease of pH in the groundwater or water phase caused by the oxidant release agent, (5) unwanted mobility issues resulting in the presence of the oxidant in places where there is no contaminant and vice versa,(6) the handling inconvenience of fine dust formation and respiratory irritation, and (7) the risk of reaction between organic gelling agent and oxidant or oxidant release agent. More specifically, this invention seeks to provide an improved composition for controlled-release of oxidants, more particularly magnesium peroxide and calcium peroxide. Both of these peroxides tend to becovered by an un reactive coating of transition metal hydroxide around the reactive oxidant-producing powder grain in contact with water. It is hence a purpose of this invention to overcome this auto-inhibition of the prolonged oxidant release.

The present invention further seeks to provide a controlled slow oxidant release system for reactive and unstable agents like hydrogen peroxide and persulfate that can readily be injected and pumped.

Briefly, the aim is to provide a soil remediation product that should be a stable, injectable and pumpable source of reactive oxidant when in contact with water, under adjustable kinetics.

Many of the current remediation products for contaminated soil, sediments and (ground) water are powders or liquids. These are rather mobile compounds which are easily washed out of the initial spot of injection with the moving (ground)water. It has been found that a gel-like product comprising the oxidant release agent and being chemically inert to the oxidant and therefore not reacting with peroxides or other strong oxidants would solve this problem by keeping the oxidant release agent in its undiluted form and keeping it at the place where it is required.

### Brief Description of the Invention

According to a first aspect, the present invention thus concerns a reactive oxidant release agent, incorporated into an inorganic viscous gel so as to ensure a slow and controlled release of oxidant. More specifically, it consists in a gel composition comprising:
- 5% to 75% w/w of an inorganic gelling agent other than silica based gelling agents,
- 0.5 to 60 % w/w, preferably 0.5 to 30% w/w, of an inorganic oxidant release agent, and
- at least 20 % w/w of water,
the inorganic gelling agent being selected such that its oxidation stage is essentially not increased by the inorganic oxidant release agent.

Under the term gel, it is herewith understood that the composition should show a viscosity of at least 150 cP. A content of gelling agent below 5% w/w leads to a composition that in most cases shows too low a viscosity. Beyond 75% gelling agent, the composition is too tough to be suitable for the uses according to the invention.

The inorganic gelling agent is advantageously selected from alkaline earth metal salts of phosphate, preferably calcium phosphate or magnesium phosphate.

As far as the oxidant or oxidant release agent is concerned, lower concentrations from 0.5 to 3 or 5 % w/w may be suitable in disinfection and wound healing applications, while higher concentrations beyond 5% w/w may be more suitable for soil and sediment remediation and (ground) water treatment.

The content of water essentially determines the viscosity of the gel. A minimum of water is required for a gel showing at least 150 cP viscosity. The person skilled in the art will control the water content of the gel such as to obtain a gel suitable for the applications as envisaged and complying with the aim of the invention that is the provision of a stable, injectable and pumpable gel that is capable of releasing oxidant when in contact with water.

The composition may further comprise 0,5 to 10% w/w, preferably 2 to 5 % w/w, more preferably 3% w/w, of a stabilizing agent. Such stabilizing agents are known per se and may be selected from Dequest ® Phosphonates (by Thermphos), 1,10-phenanthroline, 8-hydroxyquinoline, citric acid, nitrilotriacetic acid, and ethylenediaminetetraacetic acid. These stabilizing agents suitably protect the oxidant release agent from decomposition or reduction by environmental conditions such as light or other factors.

The oxidant release agent is advantageously selected from hydrogen peroxide, sodium persulfate, potassium persulfate, sodium permanganate, potassium permanganate, calcium peroxide, carbamide peroxide, magnesium peroxide, sodium percarbonate, peracetate and sodium perborate.

According to a further aspect of the invention, there is provided herein a method for producing a gel composition as described above, comprising
- preparing a first solution of a dibasic phosphate salt in water comprising dissolved oxidant release agent;
- preparing a second solution of an alkaline earth metal salt in water comprising dissolved oxidant release agent,
- mixing both, first and second solutions, in a molar ratio of alkaline earth metal to phosphate of 0.5 to 1 to 4 to 1, and
- concentrating to form a gel as defined above.

The stabilizing agent may be added to either or both solutions. One may also add stabilizing agent or oxidant after gel formation. Preferably, the dibasic phosphate salt is disodium phosphate and the alkaline earth metal salt is selected from a calcium salt, preferably calcium chloride, or a customary magnesium salt.

According to a preferred embodiment of the invention, the gel is concentrated to 90 to 20 % water, by a concentration step known per se. One may also drive the concentration further and dry up to obtain a powder suitable for certain applications.

The gels of the invention have shown to be particularly suitable for use in soil, sediment or (ground)water remediation, and the invention thus particularly relates to soil, sediment and (ground)water remediation products comprising a gel as described above.

Due the fact that the invention gel is of inorganic nature (except the stabilizer), it is inert to biodegradation and does not add extra oxygen demand upon injection. The incorporation of the oxidant release agent into the gel structure leads to a stable product with a slow-release of oxidant over time.

The concentrated gel has been found to be stable, that is suffering essentially no decomposition or deactivation of oxidant, during storage and handling for a suitable determined period of time, with a viscosity of at least 150 cP that is particularly suitable for pumping, mixing and injecting processes. The product becomes active once applied into e.g. aquifers, wet soil or sediment matrices, and starts to produce oxygen at a slow rate in contact with water. Catalysts such as ferrous iron, micron sized or nano sized particles of zero-valent metals or solid metal oxides/transition metal oxides can be combined with the product for a release of reactive oxidant species with a higher oxidation potential, such as hydroxyl radicals or sulphate radicals.

According to yet another aspect of the invention, there is provided herewith a powder composition suitable for the applications disclosed herein and obtained as described here above. In certain applications, powder compositions may be desirable despite some of the disadvantages same may entail. Such a powder composition according to the invention comprises an alkaline earth metal salt of phosphate with a molar ratio of alkaline earth metal ions to phosphate ions of 0.5 to 1 to 4 to 1, and an oxidant release agent in a weight ratio to the alkaline earth metal phosphate of 0.05 to 2.4.

The gels and powder compositions of the invention may also find suitable applications in the area of disinfection and wound care. More specifically, the invention provides a wound care product, such as a cream or ointment composition comprising a gel or powder as defined herein. A wound care product may also include a wound dressing bandage imbibed with a gel of the invention or including a powder of the invention.

### Detailed Description of the Invention

The present invention provides a controlled release source of reactive oxidant species in the form of an inorganic viscous gel, for soil and sediment remediation. As mentioned before, the gel of the present invention comprises
- 5% to 75% w/w of an inorganic gelling agent other than silica based gelling agents,
- 0.5 to 60 % w/w, preferably 1 to 30% w/w, of an inorganic oxidant release agent, and
- at least 20 % w/w of water.

According to a preferred embodiment of the invention, the gelling agent may be present in an amount of 10 to 55 % w/w, According to a more preferred embodiment, the gelling agent is present in an amount of 10 to 40 % w/w, or even more preferably 10 to 30 % w/w.

The inorganic gelling agent is advantageously selected from alkaline earth metal salts of phosphate, preferably is calcium phosphate or magnesium phosphate.

Particularly good results in soil remediation applications have been achieved with 20 to 25 % w/w calcium phosphate gelling agent in the gel composition.

The oxidant release agent is advantageously selected from hydrogen peroxide, sodium persulfate, potassium persulfate, sodium permanganate, potassium permanganate, calcium peroxide, carbamide peroxide, magnesium peroxide, sodium percarbonate, peracetate and sodium perborate. According to a preferred embodiment, the inorganic oxidant or oxidant release agent is present in an amount of 0.5 to 45 % w/w, more preferably from 0.5 to 30 % w/w. Advantageously, the oxidant release agent is present in a ratio to the alkaline earth metal phosphate of 0.3 to 2.4.

The invention provides the combination of alkaline earth metal ions, such as calcium or magnesium ions, and phosphate ions present in two separate solutions, in a molar ratio of alkaline earth metal to phosphate of 0.5 to 1 to 4 to 1: both ions are dissolved into a concentrated aqueous solution of an oxidant, such as an aqueous hydrogen peroxide solution. Both solutions are mixed at a certain ratio and concentration, until a viscous structure is obtained. If calcium ions are desired, these can be delivered from calcium chloride by dissolution into an aqueous solution of oxidant, e.g. an aqueous solution of hydrogen peroxide; whereas the phosphate can be delivered from disodium phosphate by dissolution into an aqueous solution of oxidant, e.g. an aqueous solution of hydrogen peroxide. After mixing both solutions, a gel is obtained after a concentration step, such as filtering the resulting liquid over a cellulose filter or other means of solid-liquid separation. The gelling agent may be present in an amount ranging from 5 to 75%. The resulting gel is inorganic and easily dispersible in water. The oxidant release agent utilized in the gel is present in an amount ranging from 0.5 to 60%, more preferably 0.5 to 30%, most preferably 13,75%. The oxidant release agents may be selected from or may be any combination of: hydrogen peroxide, sodium persulfate, calcium peroxide, carbamide peroxide, magnesium peroxide, sodium permanganate, potassium permanganate, sodium percarbonate, peracetate and sodium perborate. The stabilizing agent, that can be added to the gel for prolonged storage and stability, is present in an amount from 0,5 to 10%, more preferably 2 to 5%, most preferably 3% and may be selected from Dequest ®phosphonates, 1,10-phenanthroline, 8-hydroxyquinoline, citric acid, nitrilotriacetic acid, and ethylenediaminetetraacetic acid.

The combination of the above mentioned elements provides a viscous gel with a high concentration of oxidant release agent. Substantially no free oxidant (e.g. peroxide or persulphate) is measured in the gel, indicating an incorporation of the reactive oxygen species into the inorganic gel matrix (e.g. Ca₃(PO₄)₂.xH₂O₂).

Whenever a oxidant release agent is in the powdered form and is soluble in a gel matrix, the oxidant release agent can be mixed with the viscous inorganic gel for applications where additional, instant oxidative power is needed.

The slow release gel composition of the invention can be applied in soil or sediment by high pressure injection, at slightly elevated pressure or by percolation at atmospheric pressure. The viscosity of the gel can be modified by controlling the dewatering step at the end of the production process, e.g. by exposing the gel to increased temperature after production (e.g. 60 °C during 5 to 10
hours). The gel can be further dried (e.g. in an air flow) to an oxidant releasing powder.

As described above such powders may find suitable applications in soil and sediment remediation in certain cases and may still be preferred to gel compositions, despite certain disadvantages they may have compared to gel compositions. The powder compositions according to the invention comprises an alkaline earth metal salt of phosphate with a molar ratio of alkaline earth metal ions to phosphate ions of 0.5 to 1 to 4 to 1, and an oxidant release agent in a weight ratio to the alkaline earth metal phosphate of 0.05 to 2.4.

The gel compositions of the invention as well as the powder compositions of the invention may also be used for disinfection and wound care. They may be used in wound care ointments or wound dressing bandages.

### Example I. Preparation of an inorganic viscous gel with hydrogen peroxide as oxidant release agent

50g Na₂HPO₄·7H₂O was dissolved in 500ml 27,5% hydrogen peroxide by magnetic stirring (ca. 1000rpm) and gentle heating (ca. 50°C) for approximately 15 minutes. Meanwhile, 500 ml 27,5% hydrogen peroxide was added to 16g anhydrous calcium chloride that dissolved immediately without further manipulation needed. When the phosphate-mixture was completely dissolved, the two clear solutions were poured together and a thicker white substance started to form immediately. To remove the excess of liquid, this mixture was put over a paper filter with a pore size of maximum 5 µm. The remaining gel on the filter was further dried to the air for a few days. This finally yielded 140g gel at pH 6,4. With these results, the oxygen capacity of the gel can be calculated to be 11,4% (w/w), i.e. the amount of oxygen that can be released from this formulation.

| Total mixed Volumes | Gel obtained | H₂O₂ capacity | pH | Gelling Agent | Water remaining in Gel |
|---|---|---|---|---|---|
| 1L | 140 g | 22.9 % | 6,4 | 16.8 % | 60.3 % |

### Example II. Preparation of an inorganic viscous gel with a stabilizing agent and hydrogen peroxide as oxidant release agent

The gel was produced according to Example I, with the difference that Dequest®FS0536N was added to the Na₂HPO₄ solution at a concentration of 6% (v/v), just prior to addition of the CaCl₂ solution. After mixing the two solutions, it was left to settle for about one hour before it was put over a filter. The obtained gel had a total weight of 200 g, showed a pH 4,4 and an oxygen
capacity of 12% (w/w). The texture was smooth and the gel did not release oxygen after14 days at 20 °C.

| Total mixed Volumes | Gel obtained | H₂O₂ capacity | pH | Gelling Agent | Water remaining in Gel |
|---|---|---|---|---|---|
| 1L | 200 g | 24.3 % | 4,4 | 11.8 % | 63.9 % |

### Example III. Preparation of an inorganic viscous gel with potassium persulfate as oxidant release agent

The gel was produced according to Example I, but the Na₂HPO₄ and CaCl₂ powders were dissolved in a 50g/L K₂S₂O₈ solution instead of hydrogen peroxide. After filtration, 320 g of gel was obtained at pH 5,8 and the calculated sulfate radical capacity was 4,6 % (w/w). The gel had a dry, granular appearance.

| Total mixed Volumes | Gel obtained | SO4 capacity | pH | Gelling Agent | Water remaining in Gel |
|---|---|---|---|---|---|
| 1L | 320 g | 3.6 % | 5,8 | 10.9 % | 85.5 % |

### Example IV. Preparation of an inorganic viscous gel with a stabilizing agent and potassium persulfate as oxidant release agent

The gel was produced according to Example III, but in order to stabilize the persulfate, Dequest®FS0536N was added to the Na₂HPO₄ solution at a concentration of 6% (v/v) prior to adding the CaCl₂ solution. After mixing the two solutions, it was left to settle for about one hour before it was put over a filter. The obtained gel was smooth and particularly suitable for injection in contaminated sites.

| Total mixed Volumes | Gel obtained | SO4 capacity | pH | Gelling Agent | Water remaining in Gel |
|---|---|---|---|---|---|
| 1L | 270 g | 4.2 % | 4.1 | 12.9 % | 82.9 % |

### Example V: Preparation of an inorganic viscous gel with hydrogen peroxide as oxidant release agent and magnesium as earth alkali metal

50g Na₂HPO₄·7H₂O was dissolved in 500ml 27,5% hydrogen peroxide by magnetic stirring (ca. 1000rpm) and gentle heating (ca. 50°C) for approximately 15 minutes. Meanwhile, 500 ml 27,5% hydrogen peroxide was added to 16g anhydrous magnesium sulphate and stirred until a solution was obtained. When the phosphate-mixture was completely dissolved, the two clear solutions were poured together and a thicker white substance started to form slowly over 12h. To remove the excess of liquid, this mixture was put over a paper filter with a pore size of maximum 5 µm after 12h. This yielded 140g gel at pH 6,1. With these results, the oxygen capacity of the gel can be calculated to be 12.3% (w/w), i.e. the amount of oxygen that can be released from this formulation.

| Total mixed volumes | Gel obtained | H₂O₂ capacity | pH | Gelling agent | Water remained in the gel |
|---|---|---|---|---|---|
| 1 L | 195 g | 24,6% | 6,1 | 10,5% | 64,9% |

### Example VI. Oxidant release aspect of the gels

The tests for oxidant release were executed in 750 mL closed receptacles. In the closed receptacles, 500 g of soil was wetted with 250 mL tap water. Hydrogen peroxide gel was obtained according to Example II and was dosed at a concentration of 20 mL/kg soil, while mixing it under the soil asa first test set-up. In a second test set-up, hydrogen peroxide gel was obtained according to Example II and was dosed at a concentration of 20 mL/kg soil, by creating local reactive zones of 1 mL of oxidant releasing gel. In a third test set-up the slow release oxidant source calcium peroxide (powder) was used at a concentration of 12 g/kg. Hydrogen peroxide (27,5%) was used at a concentration of 20 mL/kg soil in a forth experiment. A fifth set-up was used as a control with no addition of oxidant releasing components. The oxidant release was measured by measuring the dissolved oxygen over time, as shown in Table 1.

During the measurements, the Dissolved Oxygen electrode (HANNA HI9828) was injected at the same coordinates every time and the oxygen concentration was measured 5 seconds after introducing the electrode at this spot.

**Table 1. Oxygen concentration in water saturated soil samples as a function of time, for different oxidant-release compounds**

| Time (days) | Calcium phosphate . hydrogen peroxide (ppm O₂) - mixed | Calcium phosphate . hydrogen peroxide (ppm O₂) - oxygen zones | Calcium peroxide (ppm O₂) | Hydrogen peroxide (ppm O₂) | Control (ppm O₂) |
|---|---|---|---|---|---|
| 1 | 21,5 | 33,34 | 15,84 | 10,08 | 7,13 |
| 3 | 6,30 | 7,30 | 2,3 | 2,45 | 1,75 |
| 8 | 5,67 | 3,10 | 1,38 | 1,78 | 1,24 |
| 12 | 3,63 | 3,6 | 2,52 | 1,66 | 3,25 |
| 16 | 5,93 | 6,10 | 5,36 | 4,88 | 3,78 |

From the results in Table 1, it can be seen that an inorganic viscous gel comprising oxidant release agent(s) is a suitable source of continuous oxidant release over time. The following disadvantages were overcome by this invention:
- No solidification: No formation of a precipitate that could solidify the matrix
- No immediate exothermic reaction: The product has a slow-release capability because of encapsulation of the oxidant in a slow-release gel. Oxidant release is better controlled and can be varied by changing the concentration of oxidant during the production process.
- No elevated pH: pH is close to neutral
- No migration: the gel keeps the oxidant essentially in place
- No dust formation during application

The present invention is not restricted to the exemplified embodiments and the scope of protection extends to variations and modifications that fall within the scope of the claims.

## Claims

1. A gel composition comprising
- 5% to 75% w/w of an inorganic gelling agent other than silica based gelling agents,
- 0.5-60 % w/w, preferably 0.5 to 30 % w/w, of an inorganic oxidant release agent and
- at least 20 % w/w of water
the inorganic gelling agent being selected such that its oxidation stage is not substantially increased by the inorganic oxidant release agent.

2. The composition of claim 1 wherein the inorganic gelling agent is selected from alkaline earth metal salts of phosphate, more specifically calcium
phosphate or magnesium phosphate.

3. The composition of claim 1 or 2 wherein the inorganic oxidant release agent is present in an amount of 0.5 to 45 % w/w, more preferably of 0.5 to 30 % w/w.

4. The composition according to any of claims 1 to 3 wherein the oxidant release agent is selected from hydrogen peroxide, sodium persulfate, potassium persulfate, sodium permanganate, potassium permanganate, calcium peroxide, carbamide peroxide, magnesium peroxide, sodium percarbonate,
peracetate and sodium perborate, or combinations thereof.

5. The composition according to any of claims 1 or 4 further comprising 0,5 to 10% w/w, preferably 2 to 5% w/w, more preferably 3% w/w, of a stabilizing agent.

6. The composition according to claim 5 wherein the stabilizing agent is selected from Dequest ® phosphonates, 1,10-phenanthroline, 8-hydroxyquinoline, citric acid, nitrilotriacetic acid, and ethylenediaminetetraacetic acid.

7. The composition according to any of claims 1 to 6 wherein the viscosity is at least 150 cP.

8. The composition according to any of claims 1 to 7, further comprising a catalyst.

9. The composition of claim 8 wherein the catalyst is selected from ferrous iron, micron sized or nano sized particles of zero-valent metals or solid metal oxides/transition metal oxides

10. The composition of any of claims 1 to 9, having a pH comprised between 4 and 9.

11. A soil, sediment or (ground) water remediation product comprising a gel composition according to any of claims 1 to 10.

12. A disinfection or wound care product comprising a gel composition according to any of claims 1 to 11 wherein the oxidant is comprised between 0.5 and 5 % w/w.

13. A method for producing a gel composition according to any of claims 1 to 9, comprising
- preparing a first solution of a dibasic phosphate salt in water comprising dissolved oxidant release agent
- preparing a second solution of a alkaline earth metal salt in water comprising dissolved oxidant release agent,
- mixing both, first and second solutions, in a molar ratio of earth alkali metal to phosphate of 0.5 to 1 to 4 to 1, and
- concentrating to form a gel showing a viscosity of at least 150 cP..

14. The method according to claim 13 wherein a stabilizing agent is added to either one or both solutions.

15. The method according to claim 13 wherein a stabilizing agent is added after gel formation.

16. The method according to any of claims 13- 15 wherein an oxidant is further added after gel formation

17. The method according to any of claims 14 or 15 wherein the stabilizing agent is selected from Dequest ® phosphonates, 1,10-phenanthroline, 8-hydroxyquinoline, citric acid, nitrilotriacetic acid, and ethylenediaminetetraacetic acid.

18. The method according to any of claims 13 - 17 wherein the dibasic phosphate salt is disodium phosphate and the alkaline earth metal salt is a calcium salt or a magnesium salt, preferably calcium chloride.

19. The method according to any of claims 13-18 wherein the gel is dewatered to 90 to 20% water, by a concentration step.

20. The method according to any of claims 11 to 17 wherein the gel is further
dewatered and dried to a powder.

21. A powder composition obtained by the process of claim 20 and comprising an alkaline earth metal salt of phosphate with a molar ratio of alkaline earth metal ions to phosphate ions of 0.5 to 1 to 4 to 1, and an oxidant release agent in a weight ratio to the alkaline earth metal phosphate of 0.05 to 2.4.

22. A disinfection or wound care product comprising a powder composition according to claim 21 wherein the oxidant is comprised between 0.5 and 5 % w/w.
